# FASCICULE DE BREVET EUROPEEN

(11) **EP 1 468 667 B1**
(45) Date de publication et mention de la délivrance du brevet: **06.06.2007**
(21) Numéro de dépôt: 04290947.3
(22) Date de dépôt: 09.04.2004
(51) Int. Cl.: A61K 8/68, A61Q 5/06, A61Q 5/12

(54) **Procédé de traitement capillaire et utilisation du procédé pour le lissage des cheveux**
Haarbehandlungsverfahren und dessen Einsatz zum Glätten der Haare
Hair treatment method and its use for straightening the hair

(30) Priorité: 16.04.2003 FR 0304770
(43) Date de publication de la demande: 20.10.2004
(73) Titulaire: L'ORÉAL, 75008 Paris (FR)
(72) Inventeur: Sabbagh, Anne, 92500 Rueil-Malmaison (FR); Devin-Baudoin, Priscille, 92170 Vanves (FR)
(74) Mandataire: Casalonga, Axel

(56) Documents cités:
- EP-A- 0 699 430
- EP-A- 0 774 248
- EP-A- 1 277 459
- WO-A-97/15272
- PATENT ABSTRACTS OF JAPAN vol. 2000, no. 20, 10 juillet 2001 (2001-07-10) -& JP 2001 072557 A (OKA NOBUTAKA), 21 mars 2001 (2001-03-21)

## Description

La présente invention concerne un procédé de traitement capillaire et une utilisation dudit procédé pour le lissage des cheveux.

On sait que la technique la plus usuelle pour obtenir une déformation permanente des cheveux consiste, dans un premier temps, à réaliser l'ouverture des liaisons disulfures -S-S- de la kératine (cystine) à l'aide d'une composition contenant un agent réducteur adapté (étape de réduction) puis, après avoir rincé la chevelure ainsi traitée, à reconstituer dans un second temps lesdites liaisons disulfures en appliquant, sur les cheveux préalablement mis sous tension (bigoudis et autres), une composition oxydante (étape d'oxydation, dite aussi de fixation) de façon à donner finalement aux cheveux la forme recherchée. Cette technique permet ainsi de réaliser indifféremment soit l'ondulation des cheveux, soit leur défrisage ou leur décrêpage. La nouvelle forme imposée aux cheveux par un traitement chimique tel que ci-dessus est éminemment durable dans le temps et résiste notamment à l'action des lavages à l'eau ou par shampooings, et ceci par opposition aux simples techniques classiques de déformation temporaire, telles que de mise en pli.

Le problème de la technique des permanentes connues à ce jour est que leur application sur les cheveux induit à la longue une altération de la qualité de ces derniers. Les causes essentielles de cette altération de la qualité des cheveux sont une diminution de leurs propriétés cosmétiques, telles que leur brillance, et une dégradation de leurs propriétés mécaniques, plus particulièrement une dégradation de leur résistance mécanique due à un gonflement des cheveux lors du rinçage entre l'étape de réduction et l'étape d'oxydation qui peut également se traduire par une augmentation de leur porosité.

Les cheveux sont affaiblis et peuvent devenir cassants lors de traitements ultérieurs comme des brushings.

Pour résoudre ce problème d'altération de la qualité des cheveux, il est connu d'utiliser des agents conditionneurs tels que des silicones, par exemple dans la demande de brevet internationale WO 99/17719, ou un ester de diméthicone-polyol associé à un polypeptide à fonctions terminales silanol dans la demande de brevet internationale WO 00/44337, ou encore des peptides et/ou des acides aminés dans la demande de brevet internationale WO 02/41857.

Ces solutions se révèlent insatisfaisantes dans la mesure où elles ne résolvent pas totalement le problème de l'altération des cheveux et de leurs propriétés. En particulier, dans le cas d'un traitement de déformation permanente des cheveux, ces derniers présentent un toucher non satisfaisant.

Il est également connu du document JP-A-2001-072557, des procédés de traitement capillaire tels que permanentes et lissages des cheveux, utilisant une composition de traitement qui comprend un agent réducteur des cheveux, une matrice intersticielle et un lipide intercellulaire contenant un céramide, pour éviter d'obtenir des cheveux endommagés.

La Demanderesse a alors trouvé de façon surprenante qu'un procédé de traitement capillaire comprenant l'application d'une composition contenant au moins un céramide, ladite composition étant non rincée, et l'élévation de la température des cheveux à plus de 60 °C, au moyen d'un fer plat ou rond, permettait d'apporter des propriétés cosmétiques durables telles que, par exemple, un excellent lissage des cheveux, une tonicité et une régénération de la fibre capillaire, une corporisation (ou texturisation) de la fibre, et de bons résultats de démêlage et de douceur.

L'utilisation d'un fer plat ou rond permet notamment de mieux faire pénétrer le céramide dans les fibres capillaires et d'augmenter aussi sa rémanence aux shampoings.

On entend par "fer" un dispositif de chauffage portant les cheveux à une température généralement supérieure à 60 °C.

L'extrémité du fer venant en contact avec les cheveux peut avoir différentes formes. Elle peut présenter une surface plane ("fer plat") ou une surface arrondie ("fer rond").

La présente invention a donc pour objet un procédé de traitement capillaire tel que décrit ci-dessous.

La présente invention a également pour objet l'utilisation dudit procédé pour le lissage des cheveux.

D'autres caractéristiques, aspects et avantages de l'invention apparaîtront encore plus clairement à la lecture de la description et des divers exemples qui suivent.

Selon la présente invention, le procédé de traitement des cheveux comprend les étapes consistant à :
appliquer sur le cheveu une composition contenant au moins un céramide dans un véhicule cosmétiquement acceptable, puis à
élever la température des cheveux au moyen d'un fer plat ou rond, chauffant à plus de 60 °C, de préférence à une température comprise entre 60 et 220 °C, mieux encore entre 120 et 200 °C, et à rincer éventuellement les cheveux,
ladite composition contenant au moins un céramide n'étant pas rincée avant le passage du fer.

Le céramide pouvant être contenu dans ladite composition est de préférence un composé de formule (I) suivante : 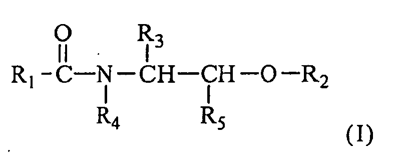 dans laquelle :
- R₁ désigne soit un radical hydrocarboné, linéaire ou ramifié, saturé ou insaturé en C₉-C₃₀, ce radical pouvant être substitué par un ou plusieurs groupements hydroxyle, ces groupements hydroxyle étant éventuellement estérifiés par un acide gras saturé ou insaturé en C₁₆-C₃₀ ;soit un radical R"-(NR-CO)-R', où R désigne un atome d'hydrogène ou un radical hydrocarboné en C₁-C₁₀ mono ou polyhydroxylé, préférentiellement monohydroxylé, R' et R" sont des radicaux hydrocarbonés dont la somme des atomes de carbone est comprise entre 9 et 30, R' étant un radical divalent ;
- R₂ désigne un atome d'hydrogène ou un radical (glycosyle)ₙ, (galactosyle)ₘ ou sulfogalactosyle, dans lesquels n est un nombre entier variant de 1 à 4 inclusivement et m est un nombre entier variant de 1 à 8 inclusivement ;
- R₃ désigne un atome d'hydrogène ou un radical hydrocarboné en C₁₆-C₂₇, saturé ou insaturé, ce radical pouvant être substitué par un ou plusieurs radicaux alkyle en C₁-C₁₄ ; R₃ peut également désigner un radical α-hydroxyalkyle en C₁₅-C₂₆ dont le groupement hydroxyle peut éventuellement être estérifié par un α-hydroxyacide en C₁₆-C₃₀ ;
- R₄ désigne un atome d'hydrogène, un radical hydrocarboné en C₁₆-C₂₇, saturé ou insaturé, ou un radical -CH₂-CHOH-CH₂-O-R₆ dans lequel R₆ désigne un radical hydrocarboné en C₁₀-C₂₆ ;
- R*₅* désigne un atome d'hydrogène ou un radical hydrocarboné en C,-C₄ mono ou polyhydroxylé.

Les céramides utilisés de préférence dans le procédé de la présente invention, répondent notamment à la formule (I) dans laquelle R₁ désigne un radical alkyle saturé ou insaturé dérivé d'acides gras en C₁₆-C₂₂ éventuellement hydroxylé ; R₂ désigne un atome d'hydrogène ; et R₃ désigne un radical linéaire saturé en C₁₅ éventuellement hydroxylé, et ils sont plus particulièrement choisis parmi
- la N-linoléoyldihydrosphingosine,
- la N-oléoyldihydrosphingosine,
- la N-palmitoyldihydrosphingosine,
- la N-stéaroyldihydrosphingosine,
- la N-béhénoyldihydrosphingosine,
- la N-2-hydroxypalmitoyldihydrosphingosine,
- la N-stéaroylphytosphingosine,
ou les mélanges de ces composés, et mieux encore parmi la N-oléoyldihydrosphingosine, la N-2-hydroxypalmitoyl-dihydrosphingosine et la N-stéaroylphytosphingosine.

Ledit céramide est contenu à une concentration se situant dans l'intervalle allant de 0,001 à 20 % en poids, de préférence de 0,01 à 10 % en poids, encore plus préférentiellement de 0,1 à 0,5 % en poids par rapport au poids total de la composition. Une concentration particulièrement préférée de l'invention est 0,5 % en poids par rapport au poids de la composition.

Le véhicule cosmétiquement acceptable utilisé dans la présente invention est constitué de préférence par l'eau ou par un mélange eau/solvant, et encore plus préférentiellement par une solution hydroalcoolique d'un alcool inférieur en C₁₋₄ tel que l'éthanol, l'isopropanol ou le butanol.

Dans le procédé selon la présente invention, on peut appliquer, en particulier, la composition contenant au moins un céramide avant ou après une composition réductrice contenant un agent réducteur, l'application de cette composition réductrice étant éventuellement suivie d'un rinçage.

Un autre mode de réalisation du procédé de l'invention consiste en ce que la composition contenant au moins un céramide contient elle-même, en outre, un agent réducteur.

Les agents réducteurs pouvant être utilisés dans le procédé de la présente invention sont notamment choisis parmi ceux généralement utilisés dans les procédés de déformation permanente des cheveux, tels que des sulfites et/ou des bisulfites de métaux alcalins ou alcalino-terreux ou d'ammonium ou, de préférence, des thiols. Parmi ces derniers, ceux les plus couramment utilisés sont la cystéïne et ses divers dérivés (notamment la N-acétylcystéïne), la cystéamine et ses divers dérivés (notamment ses dérivés acylés en C₁-C₄ tels que la N-acétyl cystéamine ou la N-propionyl cystéamine), l'acide thiolactique et ses esters (notamment le monothiolactate de glycérol), l'acide thioglycolique ainsi que ses esters, notamment le monothioglycolate de glycérol ou de glycol, le dithiodiglycolate de diammonium ou le thioglycolate d'ammonium, et le thioglycérol. On peut également mentionner les composés réducteurs suivants : les N-mercapto-alkylamides de sucres tels que le N-(mercapto-2-éthyl)gluconamide, l'acide β-mercaptopropionique et ses dérivés, l'acide thiomalique, la panthétéïne, les N-(mercaptoalkyl)ω-hydroxyalkylamides décrits dans la demande de brevet EP-A-354 835 et les N-mono- ou N,N-dialkylmercapto 4-butyramides décrits dans la demande de brevet EP-A-368 763, les aminomercaptoalkylamides décrits dans la demande de brevet EP-A-432 000 et les alkylaminomercaptoalkylamides décrits dans la demande de brevet EP-A-514 282, le mélange de thioglycolate d'hydroxy-2 propyle (2/3) et de thioglycolate d'hydroxy-2 méthyl-1 éthyle (67/33) décrit dans la demande de brevet FR-A-2 679 448.

Les composés réducteurs préférés sont notamment choisis parmi l'acide thioglycolique et ses esters, la cystéamine et la cystéine. L'agent réducteur particulièrement préféré dans l'invention est l'acide thioglycolique et ses esters tels que notamment le monothioglycolate de glycérol ou de glycol, le thioglycolate d'ammonium ou la cystéine.

La quantité d'agent réducteur est généralement comprise entre 0,1 % et 25 % en poids, de préférence entre 1 % et 15 % en poids par rapport au poids total de la composition.

La composition réductrice pouvant être utilisée dans le procédé de la présente invention présente de préférence un pH compris entre 5 et 11, et plus préférentiellement entre 6,5 et 10.

Le pH des compositions réductrices peut être éventuellement ajusté par ajout d'agents acidifiants tels que, par exemple, l'acide chlorhydrique, l'acide acétique, l'acide lactique ou l'acide borique, ou d'agents alcalinisants tels que l'ammoniac, la monoéthanolamine, le bicarbonate d'ammonium.

Les compositions utilisées dans le procédé selon l'invention peuvent contenir en outre d'autres ingrédients tels que des silicones volatiles ou non, linéaires ou cycliques, des polymères cationiques, des peptides et leurs dérivés, des hydrolysats de protéines, des cires, des agents de gonflement et de pénétration ou permettant de renforcer l'efficacité du réducteur tels que le mélange SiO₂/PDMS (polydiméthylsiloxane), le diméthylisosorbitol, l'urée et ses dérivés, la pyrrolidone, les N-alkyl-pyrrolidones, la thiamorpholinone, les alkyléthers d'alkylèneglycol ou de dialkylène-glycol tels que par exemple le monométhyléther de propylèneglycol, le monométhyléther de dipropylèneglycol, le monoéthyléther de l'éthylèneglycol et le monoéthyléther du diéthylèneglycol, des alcanediols en C₃-C₆ tels que par exemple le propanediol-1,2 et le butanediol-1,2, l'imidazolidinone-2 ainsi que d'autres composés tels que l'acide panthothénique, des agents tensioactifs anioniques, cationiques, non ioniques, amphotères ou zwittérioniques, des agents antichute, des agents antipelliculaires, des épaississants naturels ou synthétiques, associatifs ou non, des agents de suspension, des agents séquestrants, des agents opacifiants, des colorants, des filtres solaires, des vitamines ou provitamines, ainsi que des parfums et des conservateurs, et leurs mélanges.

De préférence, la composition réductrice utilisée dans le procédé de la présente invention contient au moins un agent cationique. Préférentiellement, cet agent cationique est polymérique. A titre de polymère cationique, on peut citer les produits connus sous les noms INCI, POLYQUATERNIUM 10 et HEXADIMETHRINE CHLORIDE

La composition utilisée dans le procédé de l'invention peut se présenter sous la forme d'une lotion, épaissie ou non, d'une crème, d'un gel, ou de toute autre forme appropriée.

L'application du fer peut se faire par touches séparées successives de quelques secondes, ou par déplacement ou glissement progressif le long des mèches.

A titre d'exemples de fer utilisable selon l'invention, on peut citer tous types de fer plats ou ronds et, en particulier, de manière non limitative, ceux décrits dans les brevets US 4 103 145, US 4 308 878, US 5 983 903, US 5 957 140, US 5 494 058 et US 5 046 516.

Le procédé selon l'invention peut comprendre une étape supplémentaire de pré-séchage des cheveux avec un séchoir avant l'utilisation du fer, de manière à éviter d'importants dégagements de vapeurs qui pourraient brûler les mains du coiffeur et le cuir chevelu du modèle.

Le procédé selon la présente invention peut également comporter une étape supplémentaire de fixation consistant à appliquer une composition oxydante sur les cheveux séchés, c'est-à-dire après avoir utilisé le fer.

Cette composition oxydante (ou de fixation) comprend un agent oxydant qui peut être choisi parmi le peroxyde d'hydrogène ou eau oxygénée, le peroxyde d'urée ; les bromates de métaux alcalins ; les persels tels que les perborates et persulfates ; et les enzymes telles que les peroxydases et les oxydo-réductases à deux électrons. L'utilisation du peroxyde d'hydrogène ou desdits bromates est particulièrement préférée.

La concentration d'eau oxygénée peut varier de 1 à 10 volumes, mais elle est de préférence comprise entre 6 et 8 volumes.

La concentration des bromates est généralement comprise entre 1 et 12 % en poids et celle des persels est généralement comprise entre 0,1 et 15 % en poids par rapport au poids total de la composition oxydante.

Un autre objet de l'invention est l'utilisation du procédé de la présente invention pour le lissage des cheveux.

L'exemple suivant est donné à titre illustratif de la présente invention.

### EXEMPLE

### Exemple 1

On a préparé une lotion à partir des composés suivants dans les proportions indiquées en % en poids

| | |
|---|---|
| N-oléyldihydrosphingosine | 0,25 |
| p-hydroxybenzoate de méthyle | 0,2 |
| Méthosulfate de méthyl-alkyl-alkylamidoéthyl-imidazolinium à 75 % en solution dans le propylèneglycol | 4,2 |
| Chlorure de béhényltriméthylammonium à 80 % en solution eau/isopropanol | 1,4 |
| Chlorhydrate de chlorhexidine | 0,02 |
| Eau désionisée qsp | 100 |

Cette lotion est appliquée sur cheveux venant de subir un défrisage. Après préséchage au sèche-cheveux, les cheveux sont lissés mèche par mèche à l'aide d'un fer plat chauffé à 180 °C. On obtient ainsi des cheveux disciplinés et toniques.

## Revendications

1. Procédé de traitement capillaire comprenant les étapes consistant à :
appliquer sur les cheveux une composition contenant au moins un céramide dans un véhicule cosmétiquement acceptable,
élever la température des cheveux, au moyen d'un fer plat ou rond, chauffant à plus de 60 °C, et à rincer éventuellement les cheveux,
ledit procédé étant **caractérisé en ce que** la composition n'est pas rincée avant le passage du fer.

2. Procédé selon la revendication 1, **caractérisé en ce que** la température est comprise entre 60 et 220 °C, de préférence entre 120 et 200 °C.

3. Procédé selon la revendication 1 ou 2, **caractérisé en ce qu'**il comprend une étape supplémentaire de pré-séchage des cheveux avec un séchoir avant l'utilisation du fer.

4. Procédé selon l'une quelconque des revendications précédentes, **caractérisé en ce que** le céramide est un composé de formule (I) suivante : 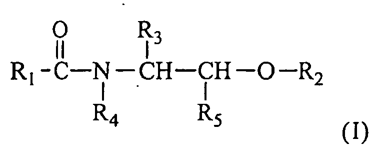 dans laquelle :
- R₁ désigne soit un radical hydrocarboné, linéaire ou ramifié, saturé ou insaturé en C₉-C₃₀, ce radical pouvant être substitué par un ou plusieurs groupements hydroxyle, ces groupements hydroxyle étant éventuellement estérifiés par un acide gras saturé ou insaturé en C₁₆-C₃₀ ; soit un radical R"-(NR-CO)-R', où R désigne un atome d'hydrogène ou un radical hydrocarboné en C₁-C₁₀ mono ou polyhydroxylé, préférentiellement monohydroxylé, R' et R" sont des radicaux hydrocarbonés dont la somme des atomes de carbone est comprise entre 9 et 30, R' étant un radical divalent ;
- R₂ désigne un atome d'hydrogène ou un radical (glycosyle)ₙ, (galactosyle)ₘ ou sulfogalactosyle, dans lesquels n est un nombre entier variant de 1 à 4 inclusivement et m est un nombre entier variant de 1 à 8 inclusivement ;
- R₃ désigne un atome d'hydrogène ou un radical hydrocarboné en C₁₆-C₂₇, saturé ou insaturé, ce radical pouvant être substitué par un ou plusieurs radicaux alkyle en C₁-C₁₄ ; R₃ peut également désigner un radical α-hydroxyalkyle en C₁₅-C₂₆ dont le groupement hydroxyle peut éventuellement être estérifié par un α-hydroxyacide en C₁₆-C₃₀ ;
- R₄ désigne un atome d'hydrogène, un radical hydrocarboné en C₁₆-C₂₇, saturé ou insaturé, ou un radical -CH₂-CHOH-CH₂-O-R₆ dans lequel R₆ désigne un radical hydrocarboné en C₁₀-C₂₆;
- R*₅* désigne un atome d'hydrogène ou un radical hydrocarboné en C₁-C₄ mono ou polyhydroxylé.

5. Procédé selon la revendication 4, **caractérisé en ce que** le céramide est un composé de formule (I) dans laquelle R₁ désigne un radical alkyle saturé ou insaturé dérivé d'acides gras en C₁₆-C₂₂ éventuellement hydroxylé ; R₂ désigne un atome d'hydrogène ; et R₃ désigne un radical linéaire saturé en C₁₅ éventuellement hydroxylé.

6. Procédé selon la revendication 5, **caractérisée en ce que** le céramide est choisi parmi :
- la N-linoléoyldihydrosphingosine,
- la N-oléoyldihydrosphingosine,
- la N-palmitoyldihydrosphingosine,
- la N-stéaroyldihydrosphingosine,
- la N-béhénoyldihydrosphingosine,
- la N-2-hydroxypalmitoyldihydrosphingosine,
- la N-stéaroylphytosphingosine,
ou les mélanges de ces composés.

7. Procédé selon la revendication 6, **caractérisée en ce que** le céramide est choisi parmi la N-oléoyldihydrosphingosine, la N-2-hydroxypalmitoyl-dihydrosphingosine et la N-stéaroylphytosphingosine.

8. Procédé selon l'une quelconque des revendications précédentes, **caractérisé en ce que** le céramide est présent en une concentration se situant dans l'intervalle allant de 0,001 à 20 % en poids, de préférence de 0,01 à 10 % en poids, et encore plus préférentiellement de 0,1 à 5 % en poids par rapport au poids total de la composition.

9. Procédé selon l'une quelconque des revendications 1 à 8, **caractérisé en ce que** l'on applique la composition contenant au moins un céramide avant ou après une composition réductrice comprenant un agent réducteur, l'application de cette composition réductrice étant éventuellement suivie d'un rinçage.

10. Procédé selon l'une quelconque des revendications 1 à 8, **caractérisé en ce que** la composition contenant au moins un céramide comprend en outre un agent réducteur.

11. Procédé selon la revendication 9 ou 10, **caractérisé en ce que** l'agent réducteur est choisi dans le groupe formé par l'acide thioglycolique et ses esters, la cystéamine et la cystéine.

12. Procédé selon la revendication 11, **caractérisé en ce que** l'agent réducteur est l'acide thioglycolique, le monothioglycolate de glycérol ou de glycol, le thioglycolate d'ammonium ou la cystéine.

13. Procédé selon l'une quelconque des revendications 9 à 12, **caractérisé en ce que** l'agent réducteur est contenu en une quantité allant de 0,1 % à 25 % en poids, de préférence de 1 % 15 % en poids par rapport au poids total de la composition.

14. Procédé selon l'une quelconque des revendications 9 à 13, **caractérisé en ce que** le pH de la composition est compris entre 5 et 11, de préférence entre 6,5 et 10.

15. Procédé selon l'une quelconque des revendications 9 à 14, **caractérisé en ce qu'**il comprend une étape supplémentaire de fixation consistant à appliquer une composition oxydante sur les cheveux secs.

16. Utilisation du procédé selon l'une quelconque des revendications précédentes, pour le lissage des cheveux.

## Claims

1. Hair treatment process comprising the steps consisting in:
applying to the hair a composition containing at least one ceramide in a cosmetically acceptable vehicle,
raising the temperature of the hair, using a flat or round iron, heating to above 60°C, and optionally rinsing the hair, the said process being **characterized in that** the composition is not rinsed out before treating with the iron.

2. Process according to Claim 1, **characterized in that** the temperature is between 60 and 220°C and preferably between 120 and 200°C.

3. Process according to Claim 1 or 2, **characterized in that** it comprises an additional step of predrying the hair with a dryer before using the iron.

4. Process according to any one of the preceding claims, **characterized in that** the ceramide is a compound of formula (I) below: 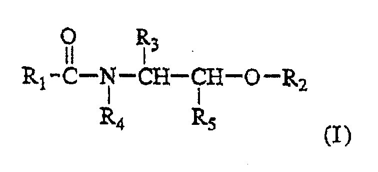 in which:
- R₁ denotes either a saturated or unsaturated, linear or branched C₉-C₃₀ hydrocarbon-based radical, this radical possibly being substituted with one or more hydroxyl groups, these hydroxyl groups optionally being esterified with a saturated or unsaturated C₁₆-C₃₀ fatty acid; or a radical R"- (NR-CO) -R' , in which R denotes a hydrogen atom or a monohydroxylated or polyhydroxylated, preferably monohydroxylated, C₁-C₁₀ hydrocarbon-based radical, and R' and R" are hydrocarbon-based radicals in which the sum of the carbon atoms is between 9 and 30, R' being a divalent radical;
- R₂ denotes a hydrogen atom or a (glycosyl)ₙ, (galactosyl)ₘ or sulphogalactosyl radical, in which n is an integer ranging from 1 to 4 inclusive and m is an integer ranging from 1 to 8 inclusive;
- R₃ denotes a hydrogen atom or a saturated or unsaturated C₁₆-C₂₇ hydrocarbon-based radical, this radical possibly being substituted with one or more C₁-C₁₄ alkyl radicals; R₃ may also denote a C₁₅-C₂₆ α-hydroxyalkyl radical, the hydroxyl group of which may optionally be esterified with a C₁₆-C₃₀ α-hydroxy acid;
- R₄ denotes a hydrogen atom, a saturated or unsaturated C₁₆-C₂₇ hydrocarbon-based radical or a radical
- CH₂-CHOH-CH₂-O-R₆ in which R₆ denotes a C₁₀-C₂₆ hydrocarbon-based radical;
- R₅ denotes a hydrogen atom or a monohydroxylated or polyhydroxylated C₁-C₄ hydrocarbon-based radical.

5. Process according to Claim 4, **characterized in that** the ceramide is a compound of formula (I) in which R₁ denotes an optionally hydroxylated saturated or unsaturated alkyl radical derived from C₁₆-C₂₂ fatty acids; R₂ denotes a hydrogen atom; and R₃ denotes an optionally hydroxylated saturated linear C₁₅ radical.

6. Process according to Claim 5, **characterized in that** the ceramide is chosen from:
- N-linoleoyldihydrosphingosine,
- N-oleoyldihydrosphingosine,
- N-palmitoyldihydrosphingosine,
- N-stearoyldihydrosphingosine,
- N-behenoyldihydrosphingosine,
- N-2-hydroxypalmitoyldihydrosphingosine,
- N-stearoylphytosphingosine,
or mixtures of these compounds.

7. Process according to Claim 6, **characterized in that** the ceramide is chosen from N-oleoyldihydrosphingosine, N-2-hydroxypalmitoyldihydrosphingosine and N-stearoylphytosphingosine.

8. Process according to any one of the preceding claims, **characterized in that** the ceramide is present in a concentration within the range from 0.001% to 20% by weight, preferably from 0.01% to 10% by weight and even more preferably from 0.1% to 5% by weight relative to the total weight of the composition.

9. Process according to any one of Claims 1 to 8, **characterized in that** the composition containing at least one ceramide is applied before or after a reducing composition comprising a reducing agent, the application of this reducing composition optionally being followed by rinsing.

10. Process according to any one of Claims 1 to 8, **characterized in that** the composition containing at least one ceramide also comprises a reducing agent.

11. Process according to Claim 9 or 10, **characterized in that** the reducing agent is chosen from the group formed by thioglycolic acid and its esters, cysteamine and cysteine.

12. Process according to Claim 11, **characterized in that** the reducing agent is thioglycolic acid, glyceryl or glycol monothioglycolate, ammonium thioglycolate or cysteine.

13. Process according to any one of Claims 9 to 12, **characterized in that** the reducing agent is contained in an amount ranging from 0.1% to 25% by weight and preferably from 1% to 15% by weight relative to the total weight of the composition.

14. Process according to any one of Claims 9 to 13, **characterized in that** the pH of the composition is between 5 and 11 and preferably between 6.5 and 10.

15. Process according to any one of Claims 9 to 14, **characterized in that** it comprises an additional fixing step that consists in applying an oxidizing composition to dry hair.

16. Use of the process according to any one of the preceding claims, for smoothing the hair.

## Patentansprüche

1. Verfahren zur Haarbehandlung, das die folgenden Schritte umfasst:
auf die Haare wird eine Zusammensetzung aufgebracht; die in einem kosmetisch akzeptablen Träger mindestens ein Ceramid enthält,
die Temperatur der Haare wird mittels eines Flach- oder Rundwerkzeugs auf mehr als 60 °C erwärmt, und
die Haare werden gegebenenfalls gespült,
wobei das Verfahren **dadurch gekennzeichnet ist, dass** die Zusammensetzung vor der Behandlung mit dem Werkzeug nicht ausgespült wird.

2. Verfahren nach Anspruch 1, **dadurch gekennzeichnet, dass** die Temperatur im Bereich von 60 bis 220 °C und vorzugsweise 120 bis 200 °C liegt.

3. Verfahren nach Anspruch 1 oder 2, **dadurch gekennzeichnet, dass** es einen weiteren Schritt umfasst, in dem die Haare vor der Verwendung des Werkzeugs mit einem Haartrockner vorgetrocknet werden.

4. Verfahren nach einem der vorhergehenden Ansprüche, **dadurch gekennzeichnet, dass** das Ceramid eine Verbindung der folgenden Formel (I) ist: 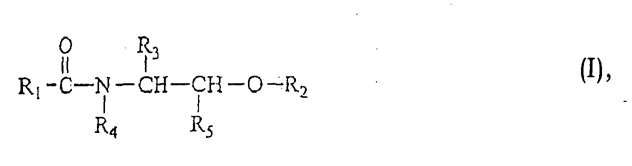 worin bedeuten:
- R₁ entweder eine geradkettige oder verzweigte, gesättigte oder ungesättigte C₉₋₃₀-Kohlenwasserstoffgruppe, wobei diese Gruppe mit einer oder mehren Hydroxygruppen substituiert sein kann, wobei die Hydroxygruppen gegebenenfalls mit einer gesättigten oder ungesättigten C₁₆₋₃₀-Fettsäure verestert sind; oder eine Gruppe R"-(NR-CO)-R', wobei R ein Wasserstoffatom oder eine ein- oder mehrfach hydroxylierte und vorzugsweise einfach hydroxylierte C₁₋₁₀-Kohlenwasserstoffgruppe ist, wobei die Gruppen R' und R" Kohlenwasserstoffgruppen bedeuten, bei denen die Summe der Kohlenstoffatome im Bereich von 9 bis 30 liegt, wobei R' eine zweiwertige Gruppe ist;
- R₂ ein Wasserstoffatom oder eine Gruppe (Glycosyl)n, (Galactosyl)m oder Sulfogalactosyl, wobei n eine ganze Zahl von 1 bis einschließlich 4 und m eine ganze Zahl von 1 bis einschließlich 8 bedeutet;
- R₃ ein Wasserstoffatom oder eine gesättigte oder ungesättigte C₁₆₋₂₇-Kohlenwasserstoffgruppe, wobei diese Gruppe mit einer oder mehreren C₁₋₁₄-Alkylgruppen substituiert sein kann; R₃ kann auch eine C₁₅₋₂₆-α-Hydroxyalkylgruppe bedeuten, bei der die Hydroxygruppe gegebenenfalls mit einer α-Hydroxysäure mit 16 bis 30 Kohlenstoffatomen verestert sein kann;
- R₄ ein Wasserstoffatom, eine gesättigte oder ungesättigte C₁₆₋₂₇-Kohlenwasserstoffgruppe oder eine Gruppe -CH₂-CHOH-CH₂-O-R₆, wobei R₆ eine C₁₀₋₂₆-Kohlenwasserstoffgruppe ist;
- R₅ ein Wasserstoffatom oder eine ein- oder mehrfach hydroxylierte C₁₋₄-Kohlenwasserstoffgruppe.

5. Verfahren nach Anspruch 4, **dadurch gekennzeichnet, dass** es sich bei dem Ceramid um eine Verbindung der Formel (I) handelt, worin R₁ eine gesättigte oder ungesättigte Alkylgruppe ist, die von C₁₆₋₂₂-Fettsäuren abgeleitet und gegebenenfalls hydroxyliert ist; R₂ ein Wasserstoffatom bedeutet; und R₃ eine gesättigte geradkettige Gruppe mit 15 Kohlenstoffatomen ist, die gegebenenfalls hydroxyliert ist.

6. Verfahren nach Anspruch 5, **dadurch gekennzeichnet, dass** das Ceramid ausgewählt ist unter:
- N-Linoleoyldihydrosphingosin,
- N-Oleoyldihydrosphingosin,
- N-Palmitoyldihydrosphingosin,
- N-Stearoyldihydrosphingosin,
- N-Behenoyldihydrosphingosin,
- N-2-Hydroxypalmitoyldihydrosphingosin,
- N-Stearoylphytosphinsogin
und den Gemischen dieser Verbindungen.

7. Verfahren nach Anspruch 6, **dadurch gekennzeichnet, dass** das Ceramid unter N-Oleoyldihydrosphingosin, N-2-Hydroxypalmitoyldihydrosphingosin und N-Stearoylphytosphinsogin ausgewählt ist.

8. Verfahren nach einem der vorhergehenden Ansprüche, **dadurch gekennzeichnet, dass** das Ceramid in einer Konzentration im Bereich von 0,001 bis 20 Gew.-%, vorzugsweise 0,01 bis 10 Gew.-% und noch bevorzugter 0,1 bis 5 Gew.-%, bezogen auf das Gesamtgewicht der Zusammensetzung, enthalten ist.

9. Verfahren nach einem der Ansprüche 1 bis 8, **dadurch gekennzeichnet, dass** die Zusammensetzung, die mindestens ein Ceramid enthält, vor oder nach einer reduzierenden Zusammensetzung aufgetragen wird, die ein Reduktionsmittel enthält, wobei nach dem Aufbringen der reduzierenden Zusammensetzung gegebenenfalls gespült wird.

10. Verfahren nach einem der Ansprüche 1 bis 8, **dadurch gekennzeichnet, dass** die Zusammensetzung, die mindestens ein Ceramid enthält, ferner ein Reduktionsmittel enthält.

11. Verfahren nach Anspruch 9 oder 10, **dadurch gekennzeichnet, dass** das Reduktionsmittel unter Thioglycolsäure und ihren Estern, Cysteamin und Cystein ausgewählt ist.

12. Verfahren nach Anspruch 11, **dadurch gekennzeichnet, dass** das Reduktionsmittel Thioglycolsäure, Glycerinmonothioglycolat oder Glycolmonothioglycolat, Ammoniumthioglycolat oder Cystein ist.

13. Verfahren nach einem der Ansprüche 9 bis 12, **dadurch gekennzeichnet, dass** das Reduktionsmittel in einer Menge von 0,1 bis 25 Gew.-% und vorzugsweise 1 bis 15 Gew.-%, bezogen auf das Gesamtgewicht der Zusammensetzung, enthalten ist.

14. Verfahren nach einem der Ansprüche 9 bis 13, **dadurch gekennzeichnet, dass** der pH-Wert der Zusammensetzung im Bereich von 5 bis 11 und vorzugsweise 6,5 bis 10 liegt.

15. Verfahren nach einem der Ansprüche 9 bis 14, **dadurch gekennzeichnet, dass** es einen zusätzlichen Fixierschritt umfasst, der darin besteht, eine oxidierende Zusammensetzung auf die trockenen Haare aufzutragen.

16. Verwendung des Verfahrens nach einem der vorhergehenden Ansprüche zur Glättung der Haare.
